(19) Europäisches Patentamt
European Patent Office
Office européen des brevets

(11) Veröffentlichungsnummer : **0 106 987**
**B1**

(12) # EUROPÄISCHE PATENTSCHRIFT

(45) Veröffentlichungstag der Patentschrift : **30.07.86**

(51) Int. Cl.⁴ : **A 61 K 7/13**

(21) Anmeldenummer : **83108619.4**

(22) Anmeldetag : **01.09.83**

(54) **Haarfärbemittel.**

(30) Priorität : **10.09.82 DE 3233540**

(43) Veröffentlichungstag der Anmeldung : **02.05.84 Patentblatt 84/18**

(45) Bekanntmachung des Hinweises auf die Patenterteilung : **30.07.86 Patentblatt 86/31**

(84) Benannte Vertragsstaaten : **AT BE CH DE FR GB IT LI NL SE**

(56) Entgegenhaltungen :
**FR-A- 1 318 072**
**US-A- 3 647 351**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Maak, Norbert, Dr. rer. nat.**
**Liebigstrasse 18**
**D-4040 Neuss (DE)**
Erfinder : **Flemming, Peter, Dr. rer. nat.**
**Rossbachstrasse 45**
**D-4200 Oberhausen 11 (DE)**
Erfinder : **Schrader, Dieter**
**Itterstrasse 7**
**D-4000 Düsseldorf (DE)**

**EP 0 106 987 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden normalerweise Entwicklersubstanzen und Kupplersubstanzen eingesetzt, die unter dem Einfluß von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiamin-derivate, m-Aminophenole, Phenole, Naphthole, Resorcin-derivate und Pyrazolone verwendet.

Gute Oxidationshaarfarbstoffvorprodukte müssen in erster Linie folgende Voraussetzungen erfüllen : Sie müssen bei der oxidativen Kupplung die gewünschten Farbnuancen in ausreichender Intensität ausbilden. Sie müssen ferner ein gutes Aufziehvermögen auf menschlichem Haar besitzen ohne die Kopfhaut zu stark anzufärben und sie sollen vor allem in toxikologischer und dermatologischer Hinsicht unbedenklich sein.

Aus DE-A-11 42 045 ist die Verwendung von Diaminopyridinen als Oxidationsfarbstoffvorprodukte bekannt. In DE-A-14 92 158 werden weitere Amino- und Oxypyridine zur Herstellung von Oxidationsfarbstoffen beschrieben. Die aus diesen Druckschriften bekannten Pyridinderivate ergeben jedoch Oxidationsfärbemittel von nicht befriedigender Farbtiefe und Echtheit.

Aus DE-A-16 17 831 ist ein Verfahren zum Färben von lebenden Haaren mit Pyridinfarbstoffvorprodukten bekannt, bei dem man zur Erzielung einer Farbvertiefung eine Reduktionsmittelmenge zusetzt, die größer ist als die zur Verhinderung einer vorzeitigen Oxidation von Farbstoffvorprodukten üblicherweise verwendete Konzentration. Dieses Verfahren hat den Nachteil, daß benzoide Farbstoffvorprodukte z. B. Kuppler zur Modifikation der Nuancen nicht eingesetzt werden können, da diese durch das Reduktionsmittel zu sehr abgeschwächt werden.

Bei der Suche nach besseren Oxidationshaarfärbemitteln bestand daher die Aufgabe, geeignete Oxidationsfarbstoffvorprodukte aufzufinden und miteinander zu kombinieren, die sowohl kräftige und tiefe Nuancen ergeben als auch eine hohe Stabilität gegenüber Wärme und Licht aufweisen. Außerdem sollen geeignete Oxidationsfarbstoffvorprodukte günstige toxikologische und dermatologische Eigenschaften aufweisen.

Aus FR-A-1318072 ist die Verwendung von 2,3-Diaminopyridin als Kuppler in Oxidationshaarfärbemitteln bekannt. Aus US-A-3647351 ist auch die Verwendung von 6-Methoxy-2,3-diaminopyridin als Oxidationshaarfarbstoff-Vorprodukt bekannt. Diese Verbindung führt jedoch zu Haaranfärbungen von geringerer Kaltwellechtheit, Reibeechtheit und Schweißechtheit.

Es wurde nun gefunden, daß Haarfärbemittel, die als Oxidationsfarbstoffvorprodukte 2,3-Diamino-6-methoxy-pyridine der Formel I

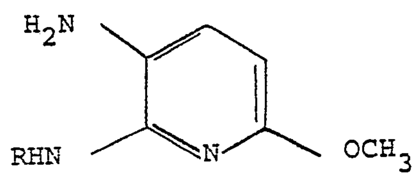

(I)

in der R eine Methyl- oder eine Hydroxyethylgruppe darstellt, in Kombination mit wenigstens einem aromatischen 1,2-Diamin, 1,4-Diamin, 2-Aminophenol und/oder 4-Aminophenol enthalten, die gewünschten Eigenschaften in optimaler Weise aufweisen.

Die 2.3-Diamino-6-methoxypyridine der Formel I können in erfindungsgemäßen Haarfärbemitteln kombiniert werden mit z. B. o-Phenylendiamin, p-Phenylendiamin, 2-Aminophenol, 4-Aminophenol und/oder Derivaten der genannten Verbindungen, die an den Stickstoffatomen Alkylsubstituenten mit 1-4 Kohlenstoffatomen oder Hydroxyalkylsubstituenten mit 2-4 Kohlenstoffatomen und/oder am aromatischen Kern Alkylsubstituenten mit 1-4 Kohlenstoffatomen und/oder Alkoxygruppen mit 1-4 Kohlenstoffatomen in der Alkylgruppe und/oder Halogenatome tragen. Beispiele substituierter aromatischer Diamine sind z. B. 2.3-Di-aminotoluol, 2.5-Diaminotoluol, 2.3-Diaminoanisol, 2.5-Diaminoanisol, N-Methyl-p-phenylendiamin, N.N-Dimethyl-p-phenylendiamin, N.N-Diethyl-2-methyl-p-phenylendiamin, N-Ethyl-N-(2-hydroxyethyl)-p-phenylendiamin, Chlor-p-phenylendiamin, N.N-Bis-(2-hydroxyethyl)-p-phenylendiamin, 2-Chlor-6-brom-p-phenylendiamin, 2-Chlor-6-methyl-p-phenylendiamin, 6-Methoxy-3-methyl-p-pheny-

lendiamin, N-(2-hydroxypropyl)-p-phenylendiamin.

Besonders ausgeprägt sind Farbtiefe und Echtheit, wenn die Verbindungen der Formel I in Kombination mit p-Phenylendiamin, p-Toluylendiamin und/oder 2.5-Diaminoanisol in den Haarfärbemitteln enthalten sind.

Die Verbindungen der Formel I sind handelsübliche Produkte.

Die erfindungsgemäßen Haarfärbemittel können neben den genannten kennzeichnenden Oxidationsfarbstoffvorprodukten noch weitere übliche Entwicklersubstanzen, Kupplersubstanzen und/oder direktziehende Farbstoffe enthalten. Solche übliche Entwicklerkomponenten sind z. B. heterocyclische Hydrazonderivate, 4-Aminopyrazolonderivate, Tetraaminopyrimidine sowie andere bekannte Aminopyridine.

Als übliche Kupplersubstanzen können zum Beispiel m-Phenylendiaminderivate, m-Aminophenole, Phenole, Resorcinderivate, Naphthole und Pyrazolone eingesetzt werden. Als direktziehende Farbstoffe kommen z. B. Nitrophenylendiamin-derivate infrage.

Die erfindungsgemäßen Haarfärbemittel zeichnen sich dadurch aus, daß äußerst brillante Haarfärbungen von hoher Stabilität gegenüber Wärme und Licht erhalten werden. Die Anwendung erhöhter Reduktionsmittel-Zusätze zu den Haarfärbemitteln ist überflüssig.

In den erfindungsgemäßen Haarfärbemitteln werden die 2.3-Diamino-6-methoxypyridine der Formel I im allgemeinen in etwa molaren Mengen, bezogen auf die verwendeten aromatischen Diamine eingesetzt. Im dem Falle, daß weitere, übliche Entwicklersubstanzen und/oder Kupplersubstanzen verwendet werden, ist es zweckmäßig, die Kupplersubstanzen in etwa molaren Mengen, bezogen auf die verwendeten Entwicklersubstanzen, einzusetzen. Wenn sich auch der molare Einsatz als zweckmäßig erweist, so ist es jedoch nicht nachteilig, einen gewissen Überschuß einzelner Oxidationsfarbstoffvorprodukte zum Einsatz zu bringen.

Es ist auch nicht erforderlich, daß die 2.3-Diamino-6-methoxypyridine oder die aromatischen Diamine sowie die gegebenenfalls zusätzlich enthaltenen Entwickler- und Kupplersubstanzen oder direktziehenden Farbstoffe einheitliche chemische Verbindungen darstellen ; vielmehr können sowohl die 2.3-Diamino-6-methoxypyridine als auch die aromatischen Diamine und die darüber hinaus enthaltenen Oxidationsfarbstoffvorprodukte und direktziehenden Farbstoffe Gemische der erfindungsgemäß einzusetzenden Verbindungen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten in Betracht.

Zur Herstellung der erfindungsgemäßen Haarfärbemittel werden die Oxidationsfarbstoffvorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder andere Zubereitungen, die für die Anwendung auf dem Haar geeignet sind. Übliche Bestandteile solcher kosmetischer Zubereitungen sind z. B. Netz- und Emulgiermittel, wie anionische, nichtionische oder ampholytische Tenside, z. B. Fettalkoholsulfate, Alkansulfonate, α-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöle, Fettsäuren, ferner Parfümöle und haarpflegende Zusätze wie zum Beispiel kationische Polymere, Proteinderivate, Pantothensäure und Cholesterin. Die Bestandteile der kosmetischen Träger werden zur Herstellung der erfindungsgemäßen Haarfärbemittel in für diese Zwecke üblichen Mengen eingesetzt ; z. B. werden Emulgiermittel in Konzentration von 0,5 bis 30 Gew.-% und Verdickungsmittel in Konzentrationen von 0,1 bis 25 Gew.-% des gesamten Färbemittels eingesetzt. Die Oxidationsfarbstoffvorprodukte werden in Mengen von 0,2 bis 5 Gew.-%, vorzugsweise 1 bis 3 Gew.-% des gesamten Färbemittels in den Träger eingemischt.

Die Anwendung der erfindungsgemäßen Haarfärbemittel kann, unabhängig von der Art der kosmetischen Zubereitung, z. B. als Creme, Gel oder Shampoo, im schwach sauren, neutralen oder alkalischen Milieu erfolgen. Bevorzugt ist die Anwendung der Haarfärbemittel in einem pH-Bereich von 8-10. Die Anwendungstemperaturen können in einem Bereich zwischen 15 °C und 40 °C liegen. Nach einer Einwirkungszeit von ca. 30 Minuten wird das Haarfärbemittel durch Ausspülen von dem zu färbenden Haar entfernt. Danach wird das Haar mit einem Shampoo nachgewaschen und getrocknet. Das Nachwaschen mit einem Shampoo entfällt, wenn ein stark tensidhaltiger Träger, z. B. ein Färbeshampoo, verwendet wurde.

Die mit den erfindungsgemäßen Haarfärbemitteln erzielbaren Färbungen haben eine hohe Brillanz sowie überlegene Wärme-, Licht-, Wasch- und Reibechtheitseigenschaften. Die nachfolgenden Beispiele sollen den Erfindungsgegenstand näher erläutern, ohne ihn jedoch hierauf zu beschränken.

## Beispiele

Die erfindungsgemäßen Haarfärbemittel wurden gegenüber solchen mit anderen Aminopyridinen

(anstelle der 2.3-Diamino-6-methoxypyridine) vergleichend in einer Haarfärbe-Cremeemulsion der folgenden Zusammensetzung geprüft :

| | |
|---|---|
| Fettalkohol $C_{12-18}$ | 10 g |
| Fettalkohol $C_{12/14}$ + 2EO-sulfat, Na-Salz (28 %ig) | 25 g |
| Wasser | 60 g |
| Aminopyridin | 0,005 Mol |
| aromatisches Amin | 0,005 Mol |
| konz. Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte wurde zunächst mit konzentrierter Ammoniaklösung der pH-Wert der Emulsion auf 9,5 eingestellt, dann wurde mit Wasser auf 100 g aufgefüllt.

Die oxidative Entwicklung der Färbung wurde mit 1 %iger Wasserstoffperoxidlösung als Oxidationsmittel durchgeführt. Hierzu wurden 100 g der Emulsion mit 10 g Wasserstoffperoxidlösung (1 %ig) versetzt und vermischt.

Die Färbecreme wurde auf ca. 5 cm lange Strähnen standardisierten, zu 90 % ergrauten aber nicht besonders vorbehandelten Menschenhaars aufgetragen und dort 30 Minuten bei 35 °C belassen. Nach Beendigung des Färbeprozesses wurde das Haar gespült, mit einem üblichen Haarwaschmittel ausgewaschen und anschließend getrocknet.

Als Aminopyridine wurden die folgenden Verbindungen eingesetzt :

A 1 : 3-Amino-2-methylamino-6-methoxypyridin (erfindungsgemäß)

A 2 : 3-Amino-2(2-hydroxyethyl) amino-6-methoxypyridin (erfindungsgemäß)

Die Verbindungen A 1 und A 2 sind Handelsprodukte. Sie wurden bezogen von der Fa. Chemische Fabrik Weyl, Mannheim.

V 1 : 2-Dimethylamino-3.5-diamino-pyridin (Vergleich)

V 2 : 2.6-Diaminopyridin (Vergleich)

V 3 : 2.5'-Diamino-bis-(2-pyridyl)-amin (Vergleich)

Als aromatisches Amin wurden die folgenden Verbindungen eingesetzt :

PPD = p-Phenylendiamin

PTD = p-Toluylendiamin

Die mit diesen Oxidationsfarbstoffvorprodukten erhaltenen Färbungen sind der Tabelle I zu entnehmen :

| Beispiel Nr. | Aminopyridin | aromat. Diamin | erhaltener Farbton |
|---|---|---|---|
| 1 | A 1 | PPD | blauschwarz |
| 2 | A 2 | PPD | blauschwarz |
| 4 | A 1 | PTD | blauschwarz |
| 5 | A 2 | PTD | blauschwarz |
| 7 | V 1 | PPD | blau |
| 8 | V 2 | PPD | blau |
| 9 | V 3 | PPD | braun |
| 10 | V 1 | PTD | blaugrau |
| 11 | V 2 | PTD | blau |
| 12 | V 3 | PTD | braun |

Die erfindungsgemäßen Haarfärbemittel gemäß Beispiel 4 und 5 wurden mit den Vergleichs-Haarfärbemitteln gemäß Beispiel 10, 11 und 12 auf die Echtheitseigenschaften gegenüber Wärme und Licht geprüft.

Prüfung der Thermostabilität

Die angefärbten Haarsträhnen wurden in einem thermostatisierten Trockenschrank 7 Tage lang bei + 45 °C gelagert. Nach dieser Wärmelagerung wurde die Farbnuance mit der einer bei + 20 °C gelagerten Probe der gleichen Anfärbung verglichen. Die Ergebnisse sind der Tabelle II zu entnehmen.

Prüfung der Lichtechtheit

Die Lichtechtheit der gefärbten Haarsträhnen wurde nach DIN 54004 (April 1966) Abschnitt 7.5.2 bestimmt. Die Methode besteht im wesentlichen darin, daß die gefärbten Haarsträhnen neben Textilproben, die mit 8 blauen, in ihrer Lichtechtheit abgestuften Typfärbungen des Lichtechtheitsmaßstabes mit einer Xenonbogenlampe mit einer Farbtemperatur von 5 500 bis 6 500 °K belichtet werden. Hierzu werden die Strähnchen und Textilproben nebeneinander auf einem Karton befestigt und die Randzonen der Strähnchen und Textilproben parallel zur Längskante des Probenträgers abgedeckt. Die Belichtung wird unter häufiger Kontrolle durch Abnehmen der Schablone solange durchgeführt, bis Typ 3 des Lichtechtheitsmaßstabes zwischen dem belichteten und dem unbelichteten Teil einen gerade wahrnehmbaren Unterschied zeigt. Dann stellt man fest, ob die Proben Änderungen zeigen und bewertet diese gegebenenfalls im Vergleich zu den Änderungen der Typen 1, 2 und 3 des Lichtechtheitsmaßstabes. Dann wird weiter belichtet, bis Typ 4 des Lichtechtheitsmaßstabes zwischen dem nunmehr belichteten und dem unbelichteten Teil ebenfalls einen gerade wahrnehmbaren Farbunterschied zeigt. Dann tauscht man die Abdeckplatte durch eine größere aus, die etwa 1/3 der zuvor belichteten Fläche parallel zur Längskante abdeckt. Die Belichtung wird fortgesetzt, bis Typ 6 des Maßstabes einen eben wahrnehmbaren Farbunterschied zeigt. Die Bestimmung der Lichtechtheit erfolgt durch Vergleich der Kontraste auf den Haarsträhnchen mit den Kontrasten auf den Typfärbungen des Lichtechtheitsmaßstabes.

Die Ergebnisse dieser Lichtechtheitsprüfungen sind der Tabelle II zu entnehmen :

Tabelle II

| Beispiel Nr. | Lichtechtheitsnote | Thermostabilität |
|---|---|---|
| 4 | 4 - 5 | stabil, unverändert |
| 5 | 3 - 4 | stabil, unverändert |
| 10 | 3 | instabil, verbräunt |
| 11 | 3 | instabil, deutlich nachgedunkelt |
| 12 | 3 | instabil, deutlich nachgedunkelt |

**Patentansprüche**

1. Haarfärbemittel enthaltend Oxidationsfarbstoffvorprodukte in einem kosmetischen Träger, dadurch gekennzeichnet, daß als Oxidationsfarbstoffvorprodukte wenigstens ein 2.3-Diamino-6-methoxypyridin der Formel I

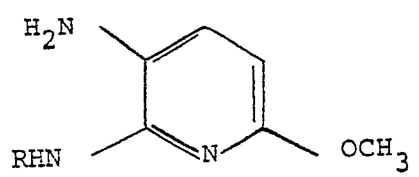

(I)

in der R eine Methyl- oder eine Hydroxyethylgruppe darstellt, in Kombination mit wenigstens einem aromatischen 1.2-Diamin, 1.4-Diamin, 2-Aminophenol und/oder 4-Aminophenol enthalten sind.

2. Haarfärbemittel nach Anspruch 1, dadurch gekennzeichnet, daß als aromatische Diamine p-Phenylendiamin, p-Toluylendiamin und/oder 2.5-Diaminoanisol enthalten sind.

3. Haarfärbemittel nach den Ansprüchen 1-2, dadurch gekennzeichnet, daß zusätzlich weitere übliche Entwicklersubstanzen und/oder Kupplersubstanzen und/oder direktziehende Farbstoffe enthalten sind.

4. Haarfärbemittel nach den Ansprüchen 1-3, dadurch gekennzeichnet, daß der Gehalt an Oxidationsfarbstoffvorprodukten 0,2 bis 5,0 Gewichtsprozent des genannten Färbemittels ausmacht.

**Claims**

1. Hair dyes containing oxidation dye precursors in a cosmetic carrier, characterized in that they contain as oxidation dye precursors at least one 2,3-diamino-6-methoxypyridine corresponding to the following formula

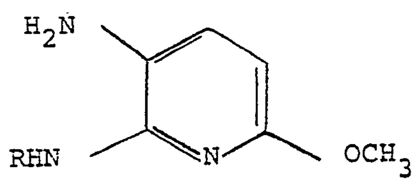

(I)

in which R is a methyl or hydroxyethyl group, in combination with at least one aromatic 1,2-diamine, 1,4-diamine, 2-aminophenol and/or 4-aminophenol.

2. Hair dyes as claimed in Claim 1, characterized in that they contain p-phenylenediamine, p-tolylenediamine and/or 2,5-diaminoanisole as aromatic diamines.

3. Hair dyes as claimed in Claims 1 and 2, characterized in that they additionally contain other standard developer substances and/or coupler substances and/or substantives dyes.

4. Hair dyes as claimed in Claims 1 to 3, characterized in that the content of oxidation dye precursors makes up from 0.2 to 5.0 % by weight of the dye.

**Revendications**

1. Composition pour la teinture des cheveux contenant des précurseurs de colorants d'oxydation sur un support cosmétique, caractérisée en ce que comme précurseurs de colorants d'oxydation, sont contenus au moins une 2,3-diamino-5-métoxy-pyridine répondant à la formule 1 :

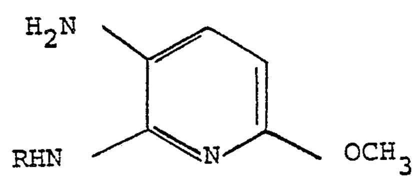

(I)

où R représente un groupe méthyle ou un groupe hydroxyéthyle, en combinaison avec au moins une diamine aromatique, 1,2-diamine, 1,4-diamine, du 2-aminophénol et/ou du 4-aminophénol.

2. Composition pour la teinture des cheveux suivant la revendication 1, caractérisée en ce qu'elle contient, comme diamine aromatique, de la p-phénylènediamine, p-toluylènediamine et/ou du 2,5-diaminoanisol.

3. Composition pour la teinture des cheveux suivant les revendications 1 et 2, caractérisée en ce qu'elle contient supplémentairement d'autres substances de développement et/ou substances de copulation et/ou des colorants montant directement.

4. Composition pour la teinture des cheveux suivant les revendications 1 à 3, caractérisée en ce que la teneur en précurseurs de colorants d'oxydation représente 0,2 à 5 % en poids du produit tinctorial mentionné.